# EUROPEAN PATENT SPECIFICATION

(11) **EP 0 551 750 B1**
(45) Date of publication and mention of the grant of the patent: **02.05.1997**
(21) Application number: 92311561.2
(22) Date of filing: 17.12.1992
(51) Int. Cl.: C12N 15/55, C12N 1/21, C12P 35/00

(54) **Recombinant DNA compounds and expression vectors encoding para-nitrobenzyl esterase activity from bacillus**
Rekombinante DNS und Expressionsvektoren die für Para-nitrobenzyl-Esterase Aktivität aus Bacillus kodieren
ADN recombinant et vecteurs d'expression codant pour l'activité para-nitrobenzyl esterase de Bacillus

(30) Priority: 20.12.1991 US 811096
(43) Date of publication of application: 21.07.1993
(73) Proprietor: ELI LILLY AND COMPANY, Indianapolis, Indiana 46285 (US)
(72) Inventor: Cantwell, Cathleen Alice, Indianapolis, Indiana 46254 (US); Hodges, Roland Lamar, Indianapolis, Indiana 46205 (US); McGilvray, Derek, Martinsville, Indiana 46151 (US); Queener, Stephen Wyatt, Indianapolis, Indiana 46208 (US); Swartling, James Richard, Indianapolis, Indiana 46220 (US); Zock, Joseph Martin, Greenwood, Indiana 46143 (US)
(74) Representative: Hudson, Christopher Mark

(56) References cited:
- EP-A- 0 549 264
- US-A- 3 972 774
- JOURNAL OF ANTIBIOTICS vol. 29, no. 2 , February 1976 , TOKYO JP pages 121 - 124 D.R. BRANNON ET AL. 'De-esterification of cephalosporin para-nitrobenzyl esters by microbial enzymes'
- FASEB JOURNAL vol. 6, no. 1 , 1 January 1992 , BETHESDA, MD US page A332 Y-R.CHEN ET AL. 'Purification and properties of p-nitrobenzyl esterase from Bacillus subtilis'

## Description

This invention relates to the field of recombinant DNA technology. In particular, it relates to DNA compounds encoding the enzyme *para*-nitrobenzyl-esterase (PNB-esterase) from the genus *Bacillus*. Esters are commonly employed intermediates in the synthesis of cephalosporin and 1-carbacephalosporin antibiotics in free acid form. The preparation of cephalosporins is taught broadly by Chauvette, U.S. Pat. No. 4,064,343. The preparation of 1-carbacephalosporins is taught broadly by Christensen *et al*., in U.S. Pat. No. 4,226,866, Munroe, in U.S. Pat. No. 4,791,106, and Hirata *et al*., U.S. Pat. No. 4,708,956.

The PNB-ester function is generally employed to block or protect the acidic carboxylic acid function in the molecule while reactions at other sites of the molecule are carried out. For example, Garbrecht, U.S. Pat. No. 3,632,850, describes the use of the *p*-nitrobenzyl ester group in the synthesis of cephalexin. In the first step of the synthesis, this ester is cleaved via hydrogenolysis under acidic conditions. In U.S. Pat. No. 3,781,282, Garbrecht describes the de-esterification of *p*-nitrobenzyl esters of cephalosporins with zinc and acid in an amide-type solvent, such as dimethylformamide. Jackson, U.S. Pat. No. 3,799,924, describes the removal of the *p*-nitrobenzyl ester group of cephalosporin esters by treatment with sodium or potassium dithionite at a pH above about 7. Hatfield, U.S. Pat. No. 4,091,214, describes a process for de-esterifying esters of cephalosporin compounds which comprises a reductive cleavage employing an inert solvent with zinc and an α-hydroxycarboxylic acid. Hirata *et al*., U.S. Pat. No. 4,708,956, describes methods for removal of *para*-nitrobezyl protecting groups from 1-carbacephalosporin compounds. Attendant with these procedures is the high cost of recycling solvents and the potential problem of pollution caused by organic solvents.

An enzymatic method for removal of PNB blocking groups used in the synthesis of cephalosporin and 1-carbacephalosporin antibiotics would have distinct advantages. Such a reaction, proceeding under mild conditions, could be completed in an aqueous reaction mixture without the use of organic solvents and metallic catalysts. Brannon, U.S. Pat. No. 3,972,774, described a process for the removal of the PNB-ester from cephalosporin esters which comprises reacting the ester with a crude preparation derived from a microorganism of the genus *Bacillus*. However, the enzyme responsible for this cleavage was not isolated.

It has not been possible to use the isolated PNB esterase enzyme isolated from *Bacillus* in an industrial scale process, because of the low level of the enzyme produced by *Bacillus subtilis*. The present invention remedies this limitation by providing recombinant DNA compounds and vectors encoding a *para*-nitrobenzyl esterase from *Bacillus subtilis*. The newly isolated DNA sequence provided herein can be used to produce PNB esterase activity in large amounts in recombinant host cells. This PNB esterase is useful for the removal of PNB ester blocking groups used in the manufacture of cephalosporin and 1-carbacephalosporin antibiotics.

This invention also provides an enzymatic cleavage process for the removal of the *p*-nitrobenzyl group from cephalosporin esters or 1-carbacephalosporin esters to provide the corresponding cephalosporin acid or 1-carbacephalosporin acid. The process comprises reacting a cephalosporin ester or 1-carbacephalosporin ester preferably in an aqueous medium, at 25° to 40°C, with the PNB esterase produced by recombinant means, and recovering the resulting cephalosporin acid or 1-carbacephalosporin acid. The process has particular value in the preparation of cefaclor and loracarbef when the 4-carboxylic group of these antibiotics is protected during synthesis by a *para*-nitrobenzyl group.

The loracarbef nucleus free acid is a substrate for penicillin G amidase in a reaction in which phenylglycine methyl ester reacts with this compound to form loracarbef and methanol. Likewise, penicillin G amidase catalyzes conversion of phenylglycine methyl ester and cefaclor nucleus free acid or cephalexin nucleus free acid to methanol and cefaclor or cephalexin.

Host-vector systems disclosed by this invention produce PNB esterase very efficiently so that the PNB esterase can represent from about 10 to 20% of the soluble protein in the recombinant *Escherichia coli* strains. This level of gene expression for active PNB esterase, measured by removal of the blocking group from the chemical intermediate loracarbef-PNB, is approximately 190-fold greater than that in *Bacillus subtilis* NRRL B8079. Moreover, because of the high level produced, the enzyme can be purified using a procedure that is considerably simpler and less expensive than that required for purification from *Bacillus subtilis* strain NRRL B8079.

The DNA compounds of the present invention encode the PNB esterase activity in a single open reading frame (orf). Transcription of this open reading frame, followed by the translation of the resulting mRNA, yields a single polypeptide chain that possesses PNB esterase activity. The DNA compound that encodes the PNB esterase activity was isolated from *Bacillus subtilis* genomic DNA and was used in the construction of the recombinant DNA expression vectors. The PNB esterase-encoding DNA compounds of this invention can be used to construct a wide variety of expression vectors which will be useful for the production of PNB esterase.

The invention includes DNA compounds that comprise a DNA sequence encoding PNB esterase activity of *Bacillus subtilis*. Also provided are recombinant DNA vectors, and recombinant host cells which contain these recombinant DNA vectors, such as *Escherichia coli*. Specific vectors that are provided by this invention include the plasmids pNB106R and pNB106RM. These vectors are useful in a method for constructing a recombinant host cell capable of expressing PNB esterase activity, said method comprising: transforming said host cell with a recombinant DNA expression vector that comprises:
(a) a promoter and translational activating sequence that functions in said host cell; and
(b) a DNA sequence encoding PNB esterase activity of *Bacillus subtilis* positioned for the expression from said promoter and translational activating sequence.

Another useful method of this invention includes a method of using a host cell constructed by the above method, for expressing PNB esterase activity, said method comprising: culturing said host cell under conditions suitable for gene expression.

For the purposes of this invention as disclosed and claimed herein, the following items are defined.

Amp - the ampicillin resistance conferring gene; also used to denote the ampicillin resistant phenotype.

cI857 - a gene encoding a temperature sensitive repressor of the bacteriophage lambda pL promoter.

Cloning - the process of incorporating a segment of DNA into a recombinant DNA cloning vector.

Coding sequence - the sequence of DNA in a gene that encodes the amino acid residue sequence of the protein expressed from the gene.

Gene - a segment of DNA that comprises a promoter, translational activating sequence, coding sequence, and 3' regulatory sequences, positioned to drive expression of the gene product.

Genomic library - a set of recombinant DNA cloning vectors into which segments of DNA, which substantially represent the entire genome of a particular organism, have been cloned.

Hybridization - the process of annealing two single-stranded DNA and/or RNA molecules to form a double-stranded molecule that may or may not be completely base paired.

loracarbef - (7B)-7-<D-(aminophenylacetyl)amino>-3-chloro-8-oxo-1-azabicyclo<4.2.0>oct-2-ene-2-carboxylic acid.

loracarbef nucleus - trans-7-amino-3-chloro-8-oxo-1-azabicyclo<4.2.0>oct-2-ene-2-carboxylic acid monohydrochloride.

loracarbef PNB ester - 7-[(aminophenylacetyl)amino]-3-chloro-8-oxo-1-azabicyclo<4.2.0>oct-2-ene-2-carboxylic acid(4-nitrophenyl)methyl ester.

mRNA - messenger ribonucleic acid.

ORI - a plasmid or vector origin of replication, the DNA sequence that serves as an attachment or start site for DNA polymerase.

pL - leftward promoter from bacteriophage lambda.

*pnb*A - the gene encoding PNB esterase activity.

Promoter - a DNA sequence that directs or initiates the transcription of DNA.

Recombinant DNA Cloning Vector - any autonomously replicating or integrating agent, including but not limited to plasmids, comprising a DNA molecule to which one or more additional DNA molecules can be added.

Recombinant DNA Expression Vector - any autonomously replicating or integrating agent, including but not limited to plasmids, comprising a promoter and other regulatory sequences positioned to drive expression of a DNA segment that encodes a polypeptide or RNA.

Recombinant DNA sequence - any DNA sequence, excluding the host chromosome from which the DNA is derived, which comprises a DNA sequence which has been isolated, synthesized, or partially synthesized.

Restriction Fragment - any linear DNA molecule generated by the action of one or more enzymes.

rop - DNA region encoding a plasmid copy control element derived from *Escherichia coli*.

TetR - the tetracycline resistance conferring gene; also used to denote the tetracycline resistant phenotype.

Transcription activating sequence - a promoter.

Transcription terminator - a DNA sequence that signals the termination of transcription of DNA by RNA polymerase.

Transformant - a recipient host cell that has undergone transformation.

Transformation - the introduction of DNA into a recipient host cell that changes the genotype of the recipient cell.

Translational activating sequence - a regulatory DNA sequence that, when transcribed into mRNA, promotes translation of mRNA into protein.

The restriction enzyme and function maps presented in the drawings are approximate representations of the recombinant DNA vectors discussed herein. The restriction site information is not exhaustive, there may be more restriction enzyme sites of a given type than are actually shown on the map.

Figure 1 is a restriction enzyme site and function map of plasmid pNBE1.

Figure 2 is a restriction enzyme site and function map of plasmid pNB106R.

Figure 3 is a restriction enzyme site and function map of plasmid pNB106RM.

The present invention comprises isolated DNA compounds and recombinant DNA cloning and expression vectors that encode the PNB esterase of *Bacillus subtilis*. The sequence of the *B*. *subtilis* PNB esterase-encoding DNA has been designated as SEQ ID NO: 1. SEQ ID NO: 1, as well as all other SEQ ID NOs, is provided in the SEQUENCE LISTING.

The amino acid sequence of the PNB esterase encoded by the SEQ ID NO: 1 is designated as SEQ ID NO: 2.

SEQ ID NO: 1 can be conventionally synthesized by the modified triester method using fully protected deoxyribonucleotide building blocks. Such synthetic methods are well known in the art and can be carried out in substantial accordance with the procedure of Itakura *et al.*, 1977, *Science* 918:1056, and Crea *et al.*, 1978, *Proc. Nat. Acad. Sci. USA* 75:5765. In addition, an especially preferred method is disclosed by Hsiung *et al*., 1983, *Nucleic Acid Research* 11:3227, and Narang *et al*., 1980, *Methods in Enzymology* 68:90. In addition to the procedures referenced above, SEQ ID NO: 1 can be synthesized using automated DNA synthesizers, such as the ABS (Applied Biosystems, 850 Lincoln Centre Drive, Foster City, CA 94404) 380A DNA Synthesizer. SEQ ID NO: 1 can also be generated by the polymerase chain reaction. See, e.g., U.S. Pat. Nos. 4,800,159 and 4,683,202 and European Patent Publication No. 0258017, published March 2, 1987.

SEQ ID NO: 2 can be encoded by a multitude of different DNA sequences because most of the amino acid residues are encoded by more that one DNA triplet. Because these alternate DNA sequences would encode the same amino acid sequence of this invention, the present invention further comprises these alternate DNA sequences.

As one of ordinary skill in the art will recognize, the present invention allows one to change the codons of the PNB esterase gene at will. Given the DNA sequence for the PNB esterase gene, procedures well known in the art can be used to generate mutant PNB esterase enzymes that vary from the natural enzyme at any number of amino acid residue positions. Such mutant enzymes would be encoded by mutant PNB esterase coding sequences, including sequences in which amino acid codons have been deleted from or inserted into the natural PNB esterase coding sequence. Such mutant enzymes are within the scope of the present invention, because even if one cannot absolutely predict whether a given mutation will destroy activity of the encoded PNB esterase, one need merely express the mutant sequence via the procedures provided herein to ascertain the effect on PNB esterase activity.

The present invention is not limited to the particular vectors exemplified herein. Instead, the present invention comprises DNA compounds that encode the PNB esterase activity of *Bacillus subtilis*. The DNA compounds of the present invention can be used to construct expression vectors that drive expression of esterase activity in any host cell in which the expression vector replicates or integrates and in which the promoter and translational activating sequence are functional.

Therefore, the present invention comprises any *Escherichia coli* plasmid or other vector that drives expression of PNB esterase activity in *E. coli*. The present invention comprises vectors that drive expression of PNB esterase activity and utilize a replicon functional in *E. coli*, such as, for example, a replicon from such plasmids as pBR322, pACYC184, F, ColV-K94, R1, R6-5, or R100. Nor is the present invention solely limited to plasmid vectors, for the present invention also comprises other vectors that express PNB esterase activity and utilize integration or viral replication to provide for replication and maintenance in the host cell.

The present invention is not limited to a particular promoter and translational activating sequence to drive expression of the PNB esterase activity-encoding DNA. The present invention comprises the use of any promoter and translational activating sequence that function in *Escherichia coli* and are used to express PNB esterase activity in *E. coli*. Many promoter and translational activating sequences functional in *E. coli* are known and are suitable for driving expression of esterase activity in *E. coli*. Such transcriptional and translational activating sequences include, but are not limited to, the *lpp, lac, trp, tac*, λpL, and λpR promoter and translational activating sequences.

In addition, transcriptional and translational activating sequences from other organisms can be ligated to the present PNB esterase-activity-encoding DNA compounds to form expression vectors that drive expression of PNB esterase activity in the host cells in which the activating sequence functions. Although *Escherichia coli* is the host best suited for PNB esterase production and subsequent purification for *in vitro* use, vectors that drive expression of PNB esterase activity in host cells other than *E. coli* are also useful.

A vector that will increase the intracellular concentration of PNB esterase activity of a given host cell into which the vector is transformed requires the following elements: 1) a PNB esterase activity-encoding DNA compound of the present invention; and 2) a promoter and translational activating sequence that not only function in the host cell to be transformed, but also are positioned in the correct orientation and position to drive expression of the PNB esterase activity-encoding DNA. Of course, stable transformants can only be obtained if the vector replicates, either as an extrachromosomal element or integrated in the genomic DNA, in the host cell. Thus, a preferred vector contains sequences that specifically direct replication or integration of the vector in the host cell. However, the presence of such specific replication or integration sequences is not absolutely required, as non-specific integration may occur when DNA is introduced into a host cell. An PNB esterase expression vector could also comprise an antibiotic resistance-conferring gene or some other element that provides a means of selecting for host cells which contain the vector, but such selectable elements may neither be necessary nor desired when the vector integrates into the chromosomal DNA of the host cell.

By providing the coding sequence of the PNB esterase gene of *Bacillus subtilis*, the present invention provides PNB esterase expression vectors for any organism susceptible to transformation. The *Escherichia coli* PNB esterase expression vectors described herein illustrate the wide variety of expression vectors of the present invention. However, many of the preferred vectors of the invention are designed to drive expression of the PNB esterase.

The following Examples are intended to assist in the further understanding of the invention. Particular materials employed, species, and conditions are intended to be further illustrative of the invention and not limiting the reasonable scope thereof. Procedures for the manipulation and analysis of DNA were performed essentially as described by Sambrook *et al.*, 1989, *Molecular Cloning:* a *Laboratory Manual*, Cold Spring Harbor Laboratory Press, Cold Spring Harbor, N.Y.

Conditions for restriction enzyme reactions were those recommended by the manufacturers (Boehringer Mannheim (BM), Indianapolis, IN; New England Biolabs (NEB), Beverly, MA; Bethesda Research Labs (BRL), Gaithersburg, MD).

### EXAMPLE 1

### Isolation of PNB Esterase Gene (pnbA) from a Genomic Library of Bacillus subtilis (NRRL B8079) DNA

### A. Description and Genotypes of Strains

*Bacillus subtilis* strain NRRL B8079 was isolated in a screen designed to identify microorganisms capable of removing the *para*-nitrobenzyl ester from cephalosporins (Brannon *et al.*, *J. Antibiotics* XXIX No. 2:121-124 1976). *B. subtilis* NRRL B-8079 has been deposited in the permanent culture collection of the Northern Regional Research Laboratory (NRRL), United States Department of Agriculture Service, Peoria, IL 61604, and is available under accession number B-8079. *Escherichia coli* K12 DH5α™ (MAX Efficiency DH5α™ Competent Cells; GIBCO BRL, Gaithersburg, MD), which is a *rec*A⁻ strain that has been developed to be highly transformable and provide a stable environment for maintenance of plasmids, was used as host strain for the *B. subtilis* strain NRRL B8079 genomic library (see Example 1J). The *rec*A+ *E. coli* K12 strain RV308, was used for high-level expression of the cloned PNB esterase gene and is a preferred host for expression of heterologous proteins in *E. coli* on an industrial scale. *E. coli* K12 RV308 has been deposited with the NRRL and is available under accession number B-15624. *E. coli* W ATCC 11105 can also be used as host for expression of heterologous proteins. This host strain can be obtained from the American Type Culture Collection (ATCC), Rockville, MD 20852, under accession number ATCC 11105.

### B. Cultivation of Strains

Trypticase®-Soy broth (TSB; Becton Dickinson Microbiology Systems) and Luria broth (L-broth; 10 g Difco Bacto-Tryptone®, 10 g NaCl, and 5 g yeast extract per liter) were used as growth medium for liquid cultures. Cultures on solid medium were grown on L-broth supplemented with 2% w/v agar (L-agar). Antibiotics were added to medium where necessary, at the following concentrations: ampicillin (50 µg/ml), and tetracycline (5 µg/ml). 5-bromo-4-chloro-3-indolyl-D-galactoside (X-gal; Sigma Chemical Co., St. Louis, MO 63178) was added to media at 20 µg/ml to detect β-galactosidase activity.

### C. Transformation of Escherichia coli K12 DH5α, E. coli K12 RV308, and E. coli W ATCC 1105

*Escherichia coli* K12 DH5α competent cells were obtained from BRL and were transformed using the manufacturer's protocol. Alternatively, 50 ml cultures of *Escherichia coli* strains DH5α, RV308 or ATCC 11105 were grown in L-broth to an OD₅₉₀ of approximately 0.5 absorbance units, and the cells were collected by centrifugation. The cell pellet was resuspended in 25 ml of cold 100 mM CaCl₂ and incubated on ice for 25 minutes. The cells were collected by centrifugation, resuspended in 2.5 ml of cold 100 mM CaCl₂ and incubated on ice overnight. Competent cells were used directly or stored at -70°C until ready for use in transformations.

To transform competent *Escherichia coli* cells, one hundred microliters of the competent cell suspension were removed from storage at -70°C and allowed to thaw on ice. Cells were gently mixed with five microliters of a solution of plasmid DNA (1 ng/µl) in a polypropylene tube, and the resulting solution was incubated on ice for 30 minutes. The tube was transferred to a 42°C water bath and incubated without shaking for 45 seconds. After this heat-shock treatment, the tube was placed on ice for 2 minutes. The tube was then removed from the ice and 0.9 ml of S.O.C. medium (2% Bacto-Tryptone®; 0.5% yeast extract; 10 mM NaCl; 2.5 mM KCl; 10 mM MgCl₂; 10 mM MgSO₄; and 20 mM glucose), pre-incubated to room temperature, was added. The cell suspension was then shaken at 225 rpm for 1 hour at 37°C and aliquots were plated on L-agar containing ampicillin. Putative transformants were picked from plates after incubation at 37°C, and their identity was confirmed by sizing their plasmid DNA using horizontal gel electrophoresis (Sambrook et al., 1989). Plasmid DNA from selected clones was characterized by restriction enzyme analysis.

### D. Plasmid DNA isolation

Plasmid DNA was isolated from *Escherichia coli* strains using a standard alkaline-SDS procedure (Sambrook *et al.*, 1989). Alternatively, plasmid DNA was isolated as follows. A portion of a transformant colony growing on L-agar containing 50 mg/ml ampicillin was transferred to one liter of L-broth containing 50 mg/ml ampicillin and incubated in an air-shaker at 37°C for about 16 hours. Cells were harvested in 500 ml bottles in a Beckmann JA-10 rotor (Beckman Instruments Inc., Fullerton, CA 92634) at 8000 rpm for 10 minutes at 4°C. The cell pellet was washed with TE, pH 8.0 (10 mM Tris-HCl, 1 mM ethylenediamine tetraacetic acid (EDTA)), collected by centrifugation, and resuspended in 50 mM Tris-HCl, pH 8.0, 25% sucrose to a total volume of 10 ml. One milliliter of 20 mg/ml lysozyme (Sigma Chemical Co., St. Louis, MO.) in 25 mM Tris-HCl, pH 8.0, was added with stirring, and the mixture was incubated on ice for 30 minutes. Four milliliters of 200 mM EDTA was added, with subsequent incubation on ice for 10 minutes, followed by the addition of 15 ml of Brij/DOC lysing solution (1% Brij 58; 0.4% deoxycholate; 50 mM Tris-HCl, pH 8.0; 60 mM EDTA). Tubes were gently inverted to mix, and incubated on ice for 15-30 minutes. Cell debris was removed by centrifugation at 18,000 rpm in a Beckman JA-20 rotor for 1 hour. Supernatant was decanted yielding approximately 30 ml, to which 150 ml of 10 mg/ml RNAse A (Sigma Chemical Co.) was added. After a 1 hour incubation at 37°C, 150 ml of 10 mg/ml Proteinase K (Boehringer Manneheim) was added followed by another 1 hour incubation at 37°C. DNA was precipitated by the addition of 1/10 volume of 3 M sodium acetate, pH 7.0, followed by 3X volumes of ice cold absolute ethanol. DNA was recovered by centrifugation in a Beckman JA-14 rotor (Beckman Instruments Inc., Fullerton, CA 92634) at 8,000 rpm for 30 minutes. The air dried pellet was resuspended in TE, pH 8.0, to a total volume of 9 ml, to which was added 9 g of cesium chloride (Boehringer Manneheim) and 0.5 ml of 10 mg/ml ethidium bromide. The resulting solution was loaded into two 5.1 ml Quik-seal tubes (Beckman Instruments Inc.) and centrifuged at 65,000 rpm in a Beckman VTi65.2 ultracentrifuge rotor for 6 hours. The plasmid band was visualized under ultraviolet light and was removed by syringe. The resulting DNA solution was extracted with salt-saturated isopropanol to remove the ethidium bromide, and dialyzed 16 hours against 1000x volumes TE, pH 8.0. DNA solutions were stored at -20°C until needed.

### E. Polymerase chain reaction (PCR) amplifications

Polymerase chain reactions were performed using a DNA Thermal Cycler™ and the Gene-Amp™ reagent kit (Perkin-Elmer Cetus, Norwalk, CT 06859) according to the instructions of the manufacturer. Thirty cycles of amplification were performed, with each cycle consisting of the following incubations: 1 min at 93°C, followed by 2 min at 40°C, followed by 4 min at 72°C. Syntheses were completed by incubation at 72°C for 10 min.

### F. Nucleotide sequence analysis

Supercoiled plasmid DNA templates for sequencing were purified from alkaline lysates of *Escherichia coli* cultures on Qiagen DNA binding columns (Qiagen, Inc., Chatsworth, CA) according to protocols provided by the manufacturer. Dideoxynucleotide chain-termination sequencing reactions were performed according to a cycle sequencing protocol (Applied Biosystems, Inc. (ABI), Foster City, CA 94404) with fluorescent dye-labelled dideoxynucleotides, supercoiled plasmid templates, and sequence specific oligonucleotide primers. Sequence reactions were analyzed on an ABI model 373A automated DNA sequencer. Nucleotide sequences were compiled, edited, and analyzed with the GCG computer programs of Devereux et al., 1985, *Nucleic Acids Res.* 12:387-395.

### G. Synthesis and End-labelling of Oligonucleotide Probes

The amino-terminal sequence of PNB esterase purified from *Bacillus subtilis* NRRL B8079 (purified by the method of Example 7) was obtained by subjecting 25 picomoles of purified PNB esterase [ca. MW = 54,000], specific activity ca. 2.2 U/mg (based on hydrolysis of loracarbef PNB ester to corresponding free acid), to analysis in an automated gas phase sequenator (Hewick *et al.*, 1981, *J. Biol. Chem.* 256:7990; Hunkapiller & Hood, 1978, *Biochemistry* 17:2124). The amino-terminal amino acid sequence is SEQ ID NO: 3.

Based on SEQ ID NO: 3 and the known codon usage for *Bacillus subtilis*, (Harwood et al., *Molecular Biological Methods for Bacillus*, John Wiley and Sons Ltd., West Sussex, England (1990)) two oligonucleotide probes were synthesized on an Applied Biosystems Inc. model 380B DNA synthesizer using β-cyanoethyl phosphoramidite chemistry, according to the manufacturer's instructions. These oligonucleotide probes are presented in the SEQUENCE LISTING as SEQ ID NO: 4 and SEQ ID NO: 5.

SEQ ID NO: 4 and SEQ ID NO: 5 were first column-purified (Oligonucleotide Purification Cartridges; Applied Biosystems, Inc.) and then end-labelled as follows. Ten picomoles of each probe were added to a 20 µl reaction mixture containing 12 µl [γ-32P] adenosine triphosphate (ATP; 5000 Ci/ml) and 8 units of T4 polynucleotide kinase in kinase buffer (50 mM Tris-HCl, pH 7.6, 10 mM MgCl₂). Following a 35 min reaction at 37°C, and a 5 minute incubation at 70°C to inactivate the kinase, the unincorporated [γ-³²p] ATP was removed from the reaction mixture using a Sephadex G-50 Nick Column (Pharmacia Inc, Piscataway, NJ 08854).

### H. Isolation of Genomic DNA from Bacillus subtilis NRRL B8079

Total DNA was isolated from a two liter culture of *Bacillus subtilis* NRRL B8079 grown to mid-logarithmic phase in TSB at 25°C for 16 hours. Cells were harvested by centrifugation and resuspended in 20 ml of buffer (50 mM Tris-HCl, pH 8.0, 50 mM EDTA). Lysis of cells was accomplished in two steps by: (i) addition of 1 mg/ml lysozyme (from egg white; Calbiochem, La Jolla, CA) and incubation of the suspension for 20 min at 37°C, and (ii) addition of 4 ml of lysing buffer (0.5% sodium dodecyl sulfate [SDS], 50 mM Tris pH 7.5, 0.4 M EDTA, 1 mg/ml Proteinase K) and incubation at 50°C for 30 min. The DNA was purified by two phenol extractions followed by one chloroform extraction, and then recovered by precipitation with ethanol and spooling on a glass rod. RNA was removed from the preparation by gently resuspending the DNA in 40 mM Tris-HCl buffer, pH 7.5, containing 1 mM EDTA and 0.2 mg/ml ribonuclease (from bovine pancreas; Sigma Chemical Co.) and then incubating the preparation at 25°C for 30 min. Further purification was accomplished by re-extraction with phenol and chloroform, precipitation with ethanol, and resuspension in TE, pH 7.5.

### I. Southern Hybridizations of EcoRI Restriction Fragments Using Oligonucleotide Probes

Genomic DNA from *Bacillus subtilis* NRRL B8079 was digested with *Eco*RI and subjected to electrophoresis on a 1% agarose-TBE gel (Sambrook et al., 1989). The size-fractionated DNA was then transferred to Hybond-N+ nylon membranes (Amersham, Arlington Heights, IL, U.S.A.) by Southern blotting (Sambrook et al., 1989), and cross-linked to the matrix by treatment with ultraviolet light for 5 min. After pre-hybridization of the DNA at 70°C for 1-2 hr in hybridization buffer containing 6X SSC (1X SSC, pH 7.0, contains 0.15 M sodium chloride, 0.015 M sodium citrate), 5X Denhardt's solution (1X Denhardt's solution contains 0.2 g/l Ficoll 400, 0.2 g/l polyvinylpyrollidone and 0.2 g/l bovine serum albumin [Fraction V]), 0.25% SDS, and 20 µg/ml calf thymus DNA, fragmented by treatment with ultrasound. Fresh hybridization buffer containing [³²P]-labelled oligonucleotide (prepared as described in Example 1G) was then added to produce a final probe concentration of 0.5 pmoles/ml. The temperature of the incubation mixture was allowed to fall to 45°C during overnight incubation. The membranes were then washed using conditions of stringency that had been optimized for each probe. Membranes hybridized to SEQ ID NO: 4 were washed for 25 min, three times in succession, at 45°C in 4X SSC, 0.25% SDS. Membranes hybridized to SEQ ID NO: 5 were washed similarly, with 0.5X SSC, 0.25% SDS. After washing, the membranes were dried at room temperature and exposed to film.

Using the above procedure, a band representing EcoRI fragments of approximately 6 kb in size was shown to hybridize to SEQ ID NO: 4 and SEQ ID NO: 5.

### J. Construction of an Enriched Library of EcoRI DNA Fragments and Isolation of pNBE1

*Bacillus subtilis* NRRL B8079 genomic DNA was completely digested with EcoRI and subjected to electrophoresis in a horizontal 1.2% agarose gel (in 1X TAE buffer containing 40 mM Tris-acetate, 1 mM EDTA; 2V/cm) for 16 hr. The gel was stained in a dilute (1 mg/ml) ethidium bromide solution and the DNA bands visualized under long-wave ultraviolet light. A slice was removed from the region of the gel that corresponded to DNA of approximately 6 kb in size, and that spanned the band that had previously been shown to hybridize to SEQ ID NO:4 and SEQ ID NO:5. Elution of the DNA fragment from the gel slice was performed according to the protocol of Sambrook *et al.* (1989), with minor adjustments. Briefly, the gel slice was put into a dialysis bag with 200 µl 0.2X TAE buffer, sealed with clips, and electrophoresed in 0.2X TAE buffer for about 3 hours. The contents of the dialysis bag, including a 400 µl wash with 0.2X TAE, were mixed with 2.4 ml of low salt buffer (0.2 M NaCl; 20 mM Tris-HCl, pH 7.5; 1.0 mM EDTA) and loaded onto an ELUTIP-d column (Schleicher & Schuell, Keene, NH) prepared according to the manufacturer's protocol. After washing with low salt buffer the DNA was eluted from the column with two 400 µl volumes of high salt buffer (1.0 M NaCl; 20 mM Tris-HCl, pH 7.5; 1.0 mM EDTA) and precipitated by addition of 800 µl of ice-cold absolute ethanol followed by centrifugation at 14,000 rpm in an Eppendorf 5415C microfuge for 40 minutes. The two air-dried pellets were combined by dissolving in 20 ml TE, pH 7.5, and the solutions were stored at -20°C until ligation. The fragments were ligated into the vector pUC19 (Gibco BRL, Gaithersburg, MD) which was pre-treated by digestion with *Eco*RI followed by removal of the 5' phosphates using calf intestinal alkaline phosphatase (Calbiochem; La Jolla, CA).

The resulting plasmids were used to transform competent *Escherichia coli* K12 DH5α cells, which were then plated onto L-agar plates containing ampicillin and X-Gal. Use of plasmid pUC19 as a vector allowed the specific selection of clones containing insert DNA using blue/white selection. Transformants containing only the pUC19 vector, which includes that portion of the lacZ gene that codes for the α-peptide of β-galactosidase, produce active enzyme, detected by cleavage of X-Gal to produce a blue color. In contrast, isolates containing an insert at the *Eco*RI site disrupt the reading frame of the α-peptide, and are unable to convert the X-Gal, and thus remain white on media containing X-Gal. More than one hundred white, ampicillin-resistant colonies were selected and replated on the same agar to test phenotypic stability.

Plasmid DNA was isolated from white colonies and analyzed by gel electrophoresis. Seven colonies containing plasmid DNA of the required 9 kb size (2.686 kb pUC19 DNA plus approximately 6 kb insert DNA) were selected for further study. When tested by Southern hybridizations, using the SEQ ID NO: 4 probe, one plasmid, pNBE1, gave a strong hybridization signal. Digestion of plasmid pNBE1 with EcoRI yielded two fragments of the predicted size: one corresponding to linearized pUC19 (2.686 kb), and the other, approximately 6 kb in size, corresponding to the insert fragment.

### K. Demonstration of the direction of transcription of pnbA on plasmid pNBE1

Two PCR amplifications using pNBE1 were performed to determine the direction of transcription of *pnb*A, relative to the *lac*Z gene of pUC19, and the approximate position of the ATG transcription start site in the insert. The first amplification used the SEQ ID NO:5 oligonucleotide probe and the forward (-20) M13 DNA sequencing primer (New England BioLabs). The second amplification also used SEQ ID NO:5, but the second primer was the reverse (-24) M13 sequencing primer. Results showed that only the reaction with the (-20) forward sequencing primer generated an amplified fragment. The size of the amplified fragment was determined to be 2.0 kb. Therefore, the ATG start site of *pnb*A is located approximately 4 kilobase pairs (kb) downstream of the start of the multi-cloning site of pUC19, and is transcribed in the same direction as the *lac*Z gene.

### L. Verification of a functional pnbA gene

To determine whether the entire *pnb*A gene was present on pNBE1 and capable of being expressed in *Escherichia coli*, extracts of *E. coli* K12 DH5α cells transformed with plasmid pNBE1 were assayed for esterase activity. Cell extracts were prepared from approximately 75 ml of a culture grown to mid-logarithmic phase in L-broth. Cells were collected by centrifugation for 10 min at 8,000 rpm, washed in 10 mM sodium phosphate buffer, pH 7.0, and recentrifuged. The cell pellet was resuspended (1 g cells/4 ml buffer) and disrupted by exposure to four 30 second bursts of ultrasound (Sonicator™ Cell Disruptor Model W185F, Heat Systems-Ultrasonics Inc., Plainview, N.Y.). Cell debris was removed by centrifugation in a microfuge for 20 min at maximum speed. Because purified PNB esterase catalyzes the cleavage of *p*-nitrophenyl acetate, in addition to *p*-nitrobenzyl esters of β-lactam antibiotics, this reaction could conveniently be used for monitoring the formation of *p*-nitrophenol spectrophotometrically. The reaction mixture contained 400 µl of 0.5 M *p*-nitrophenyl acetate (in 1:99 v/v acetonitrile/water), 600 µl of 167 mM Tris-HCl, pH 7.0, and 1-20 µl of cell free extract. Formation of *p*-nitrophenol was measured spectrophotometrically by monitoring the increase in OD₄₀₅ using a Gilford Response 11 spectrophotometer. Alternatively, enzyme activity could be demonstrated qualitatively using whole cells by adding a portion of a colony to the reaction mixture using a toothpick. Assay of both whole cells and cell free extracts using these procedures demonstrated that plasmid pNBE1 contained an intact *pnb*A gene, and that the gene was expressed to produce active enzyme. No significant activity was detected in *Escherichia coli* K12 DH5α cells without plasmid, or with *E. coli* K12 DH5α cells carrying other non-hybridizing plasmids from the genomic library.

### M. Subcloning of the pnbA gene

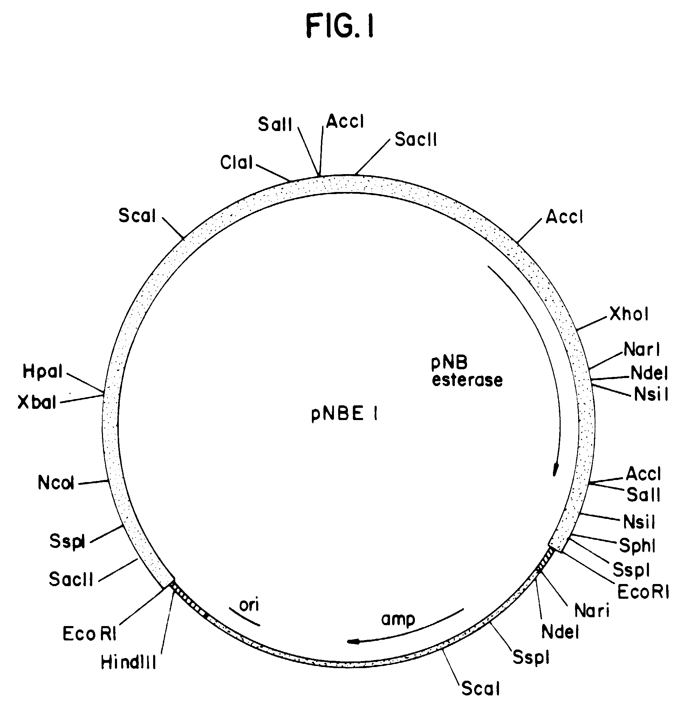

With the information obtained from the PCR experiment described in Example 1K, and results from single and double restriction enzyme digestions of pNBE1, it was possible to construct a preliminary physical map of the 6.0 kb *Eco*RI fragment. A restriction enzyme and function map of plasmid pNBE1 is presented in Figure 1. A series of subclones of pNBE1 were then made by partial digestion of the plasmid with *Hpa*II, followed by complete digestion with *Eco*RI, and ligation of the resulting *Hpa*II-*Eco*RI fragments to pUC19 digested with *Acc*I-*Eco*RI. The *Hpa*II site of the insert and the *Acc*I site of pUC19 were not regenerated by this ligation. The resulting plasmids were then transformed into *Escherichia coli* K12 DH5α cells, and ampicillin-resistant colonies were selected and tested for esterase activity. Plasmid DNA was isolated from a number of positive clones and analyzed to determine the size of the *Hpa*II-*Eco*RI inserts. The smallest insert that coded for active enzyme was 2.3 kb. The plasmid vector was designated pNBHpE2.3.

An additional subcloning experiment was performed which indicated that the *Sal*I-*Eco*RI fragment at the distal end of the 2.3 kb insert was not required for a functional pnbA gene. pNBHpE2.3 was first digested with *Hind*III and *Sal*I, ligated to pUC19 digested with *Hind*III and *Sal*I, and then transformed into *Escherichia coli* K12 DH5α cells. The plasmid from this construction, designated pNBH3S1.9, contained a 1.9 kb *Hpa*II-*Sal*I insert, and coded for active PNB esterase protein. The insert DNA from this plasmid was subcloned into pBluescript SK(-) (Stratagene, La Jolla, CA) and analyzed to determine the nucleotide sequence. The coding sequence of the PNB esterase gene from *Bacillus subtilis* is presented in the Sequence Listing as SEQ. ID. NO. 1.

### EXAMPLE 2

### High Level Expression of the pnbA Gene in Escherichia coli

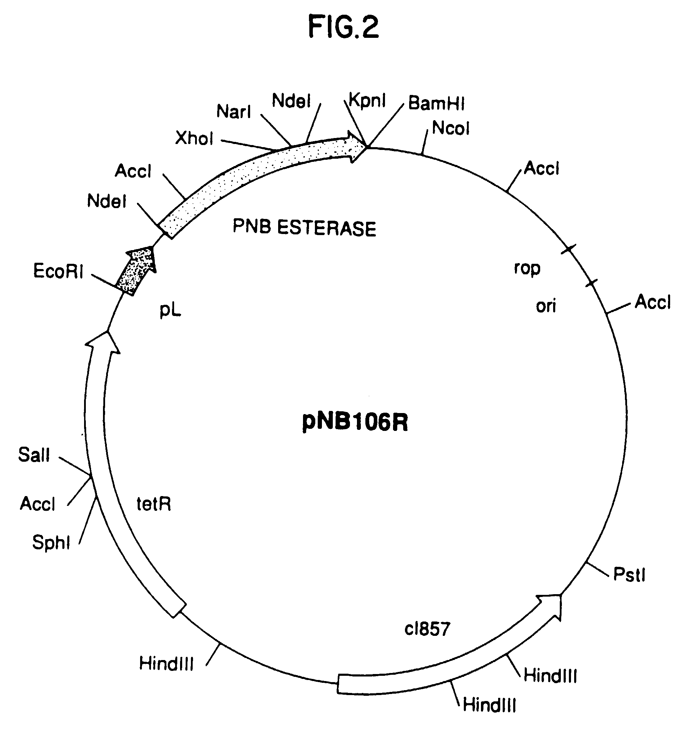

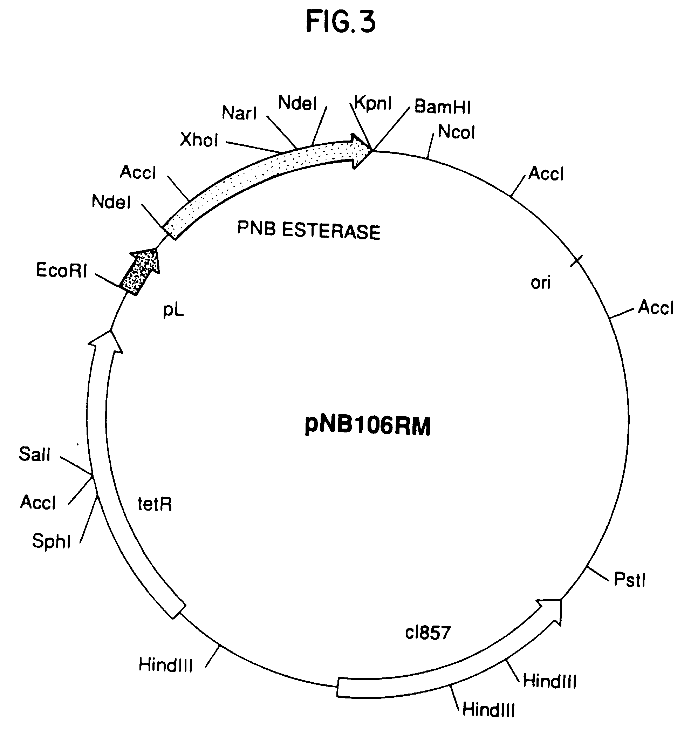

Expression of the cloned PNB esterase gene in *Escherichia coli* was improved by construction of vectors pNB106R and pNB106RM. Plasmid pNB106R contains a functional rop gene, responsible for control of plasmid copy number (Cesareni *et al.*, 1982, *Proc. Natl. Acad. Sci. USA* 79:6313). A restriction enzyme and function map of plasmid pNB106R is presented in Figure 2. Plasmid pNB106RM, which lacks the *rop* gene, was derived from pNB106 to determine the effect of plasmid copy number upon expression of the *pnb*A gene. A restriction enzyme and function map of plasmid pNB106RM is presented in Figure 3.

### A. Construction of Escherichia coli K12 DH5α/pNB106R, E. coli K12 RV308/pNB106R and E. coli W ATCC 11105/pNB106R.

Plasmid pNB106R was generated by subcloning the 1.9 kb DNA fragment from plasmid pNBH3S1.9 into the temperature inducible high level expression vector pHKY338 to provide temperature inducible expression of a heterologous gene from a modified bacteriophage lambda pL promoter. *E. coli* K12 RV308/pHKY338 was deposited with the NRRL on January 31, 1992, and is available under accession number NRRL B-18945. The plasmid contains two regions of DNA of particular importance: (i) a sequence derived from pBR322 which allows replication and maintenance of the plasmid in *Escherichia coli*, and in which the ampicillin resistance gene of pBR322 is replaced by a lambda cI857 repressor gene; and (ii) a sequence in which a modified lambda promoter, pL106 (U.S. Patent Application Serial No. 07/739,280, European Patent Application 91307325.0), situated downstream of the tetracycline resistance gene, is immediately adjacent to the open reading frame (ORF) of a kanamycin resistance gene. The promoter controls transcription of that ORF via an two cistron assembly (Schoner et al., 1990, *Methods in Enzymology* 185:94). Plasmid pHKY338 was constructed so that the open reading frame of the kanamycin resistance gene can easily be removed by digestion with *N*deI and *Bam*HI. Any ORF with an *N*deI restriction site at its ATG transcriptional start codon, and a *Bam*HI compatible restriction site downstream from its potential transcription termination sequences, can be inserted into pHKY338. The resulting inserted ORF will then be aligned correctly for expression from the pL promoter.

Because the cloned 1.9 kb *Bacillus subtilis* DNA did not contain an NdeI site at the desired location, a strategy was developed to introduce one at the ATG transcription start site of the *pnb*A ORF. The strategy involved separate constructions to generate amino-terminal and carboxy-terminal fragments of the *pnb*A gene, followed by ligation of the two fragments to a third fragment, obtained from the pHKY338 vector. Construction of the three fragments was accomplished as follows:

### i) Amino-terminal fragment.

A 261 bp fragment coding for the amino-terminus of PNB esterase, containing an *Nd*eI site adjacent and 5' to the ATG transcription start site, was synthesized using PCR technology (see Example 1E). The PCR amplification was performed using the following the two primers designated as SEQ ID NO: 6 and SEQ ID NO: 7.

SEQ ID NO.6 contains an *Nde*I restriction site. The first three bases of the primer were not matched to the cloned PNB esterase gene. The other primer, SEQ ID NO: 7, anneals approximately 25 bp downstream from the *Acc*I site located 215 bp downstream from the ATG start site. The 261 bp fragment that resulted from the amplification was treated with Proteinase K (protease Type XXVIII, from *Tritirachium album*) according to the procedure described by Crowe *et al.*, 1991, *Nuc. Acid Res.* 19:184, with minor modifications. The fragment was subsequently digested with *Nde*I and *Acc*I, phenol extracted and ethanol precipitated by standard methods to generate the 180 bp fragment used in the ligation reaction presented below,

### ii) Carboxy-terminal fragment

Because the construction strategy required use of the *Acc*I site internal to the ORF to splice the amino-terminal and carboxy-terminal fragments it was necessary to eliminate the other *Acc*I site (*Sal*I/*Acc*I) on the 1.9 kb fragment. This was accomplished in two steps: (i) digestion of pNBH3S1.9 with *Hind*III and *Sal*I to release the ORF fragment; and (ii) ligation of the released fragment to pBluescript SK(-) that had been digested with *Hind*III and *Xho*I. Insertion of the *Sal*I/*Acc*I site of the 1.9 kb fragment into the *Xho*I site of pBluescript SK(-), eliminated all three sites. Transformation of the ligation mixture into *Escherichia coli* K12 DH5α, followed by screening of putative clones and confirmation of structures by restriction digests, resulted in the isolation of pNBH3(SX)sk, which has only one *Acc*I site, located in the ORF. Another construction was needed to introduce a *Bam*HI site at the carboxy-terminus. This was accomplished by digestion of pNBH3(SX)sk with *Eco*RI and *Kpn*I, to release the ORF fragment, followed by ligation of this fragment into pUC19, also digested with *Eco*RI and *Kpn*I. Transformation of the ligation mixture into DH5α cells, followed by selection and analysis of clones, resulted in the isolation of plasmid pNBEKuc, which contained a single *Acc*I site in the ORF, and an appropriately positioned *Bam*HI site downstream from the carboxy-terminus.

### iii) Vector fragment from pHKY338

Vector for the three piece ligation reaction presented below was prepared by digestion of pHKY338 with *Nde*I and *Bam*HI to remove the kanamycin resistance gene, and gel isolation of the large restriction fragment.

A three piece ligation was performed involving the *Nde*I-*Acc*I digested PCR fragment (180 bp), the *Acc*I-*Bam*HI fragment of pNBEKuc (1.7 kb), and the *Nde*I-*Bam*HI fragment of pHKY338 (6 kb). This produced plasmid vector pNB106R, containing the entire *pnb*A ORF under control of the temperature inducible lambda pL synthetic promoter. The resulting DNA was transformed into host strain *Escherichia coli* K12 DH5α. Plasmid pNB106R was subsequently transformed into host cells *E. coli* K12 RV308 and *E. coli* W ATCC 11105.

### EXAMPLE 3

### Construction of Escherichia coli K12 DH5α/pNB106RM, E. coli K12 RV308/pNB106RM and E. coli W ATCC 11105/pNB106RM

To construct a version of pNB106R without the *rop* gene (*rop*⁻), the plasmids PNB106R and pHKY389 (*rop*⁻) were both digested with *Sph*I and *Nco*I. *Escherichia coli* K12 RV308/pHKY389 was deposited with the NRRL on October 22, 1992, and is available under accession number B-21012. The ∼4.5 kb *Sph*I-*Nco*I fragment from pHKY389, and the ∼3 kb *Sph*I-*Nco*I fragment from pNB106R were gel isolated following electrophoresis using low melting agarose. These fragments were ligated using T4 DNA ligase and standard reaction conditions at 37°C for 4 hr. Competent *E. coli* K12 DH5α cells were transformed and plated on L-agar containing tetracycline. Selected clones were screened for the expected AccI restriction enzyme pattern, and a strain carrying plasmid pNB106RM was thereby identified. Plasmid pNB106RM was subsequently transformed into strains *E. coli* K12 DH5α, *E. coli* K12 RV308, and *E. coli* W ATCC 11105 using the procedure described in Example 1.

### EXAMPLE 4

### Synthesis of PNB Esterase in Escherichia coli K12 DH5α, E. coli K12 RV308 and E. coli W ATCC 11105 Transformed with pNB106R or pNB106RM.

Synthesis of PNB esterase by cultures of *Escherichia coli* K12 DH5α, *E. coli* K12 RV308 and *E. coli* W ATCC 11105 transformed with plasmid pNB106R or pNB106RM was induced as follows. A frozen stock culture of the strain was used to inoculate L-broth containing 5 µg/ml tetracycline. After 16 hr growth at 30°C, cells were subcultured (4% v/v) into fresh L-broth plus tetracycline and grown to mid-logarithmic phase. Induction of enzyme synthesis was accomplished by raising the temperature of the culture to 40°C. The kinetics of PNB esterase synthesis was measured by sampling the culture periodically and assaying enzyme activity in cell free extracts as described in Example 1L. Cell-free extracts were also analyzed by SDS-polyacrylamide gel electrophoresis (SDS-PAGE) to allow the relative increase in the amount of PNB esterase protein to be monitored.

### EXAMPLE 5

### Purification of PNB Esterase from Recombinant Escherichia coli Strains.

The PNB esterase was purified from *Escherichia coli* K12 DH5α/pNB106R by the procedure outlined in Table 1. This procedure can be compared to the purification from *Bacillus*, presented in Example 7 and outlined in Table 2. It will be noted that in the latter case eight (8) steps were required and 2.6 mg of pure enzyme was obtained from 130,000mg of crude protein, whereas from the recombinant *Escherichia coli* strain 10 mg of pure enzyme was obtained from only 200 mg of crude protein using only 6 steps. Thus, the simpler process of purification, using recombinant *E. coli* as the source, was 3250 times more efficient than the old more complex purification process where the natural producer, *Bacillus subtilis*, was used as enzyme source.

Because the new purification procedure with recombinant *Escherichia coli* as the source of enzyme is considerably simpler, it would be less expensive to apply at large scale than the purification procedure needed for the same enzyme obtained from *B. subtilis*. This is a direct result of the significantly increased amount of PNB esterase obtained in the recombinant *E. coli* strain (ca. 3250 fold per unit crude protein).

The PNB esterase purified from recombinant *Escherichia coli* was compared to the native enzyme by molecular weight, Western blot analysis, and substrate specificity. Measurements of molecular weight gave values of 54,000 for enzyme from the two sources, and identical precipitin bands were obtained when the two enzyme preparations were tested using antibody prepared against native purified esterase. Both enzymes hydrolyzed cefaclor PNB ester, cefaclor nucleus PNB ester, loracarbef PNB ester, and loracarbef nucleus PNB ester. The native PNB esterase and the corresponding PNB esterase produced in recombinant *E. coli* strains appear functionally identical and structually the same at macroscale.

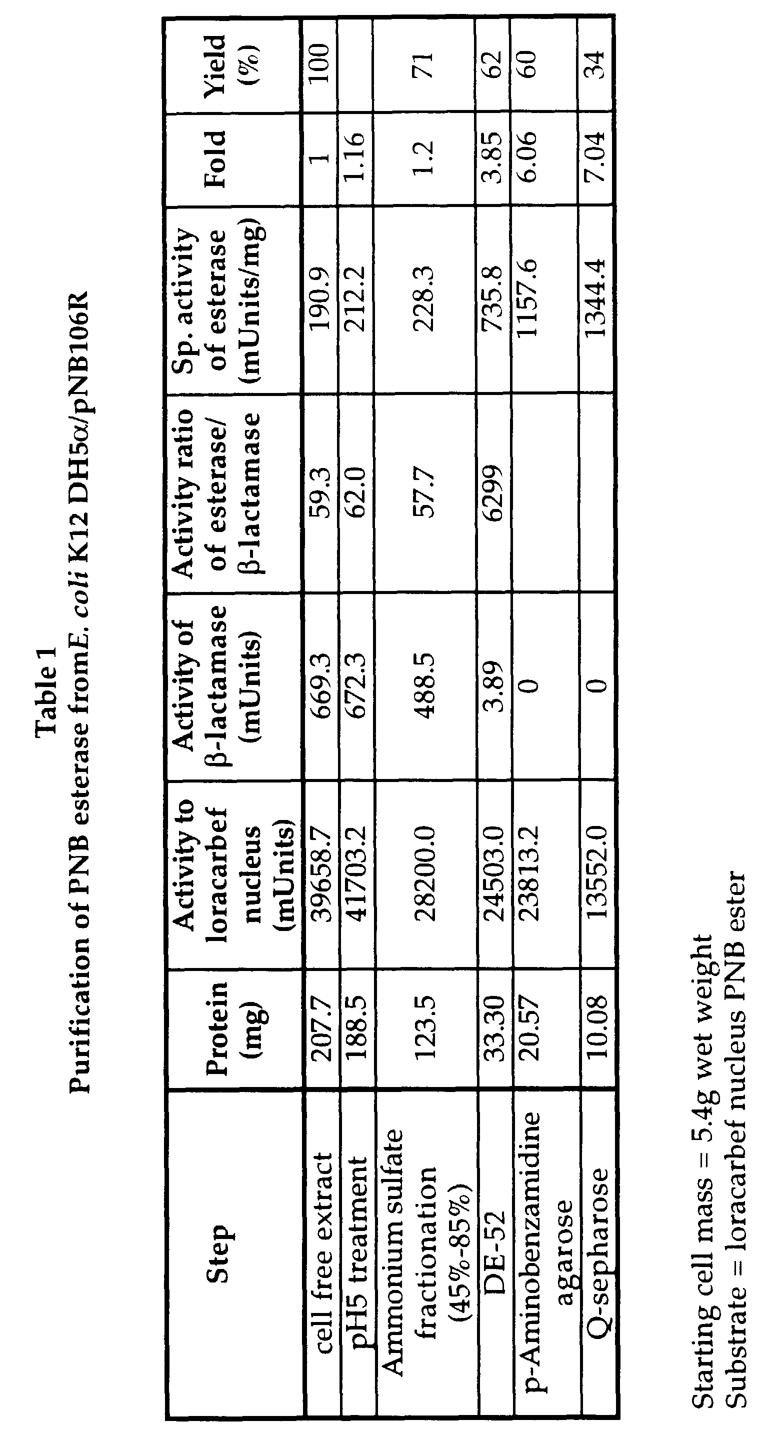

### EXAMPLE 6

### Hydrolysis of Loracarbef Nucleus PNB Ester by Partially Purified PNB Esterase

Partially purified PNB esterase (2.5 mg based on specific activity and known turn-over number of the enzyme) prepared from *Escherichia coli* K12 DH5α/pNB106R cells, and treated with N-ethyl maleimide to inactivate residual β-lactamase, was added to a suspension of loracarbef-nucleus PNB ester (2.2% w/v) in 50 mM Tris buffer, pH 8.0. The reaction mixture was maintained at 37°C for 3 hours. Sodium hydroxide solution was added periodically to maintain the pH at 8.0. After 3 hours there was a 90% yield of loracarbef-nucleus free acid, based on high performance liquid chromotagraphy (HPLC) analysis for that compound. Further incubation did not increase the yield.

### EXAMPLE 7

### Isolation and Purification of PNB Esterase from Bacillus subtilis

### A. Preparation of Cell-Free Extract

A culture of *Bacillus subtilis* NRRL B-8079 was harvested and frozen by conventional methods. The frozen *B. subtilis* cells (760 g) were thawed and homogenized in 2 liters of Buffer A (10 mM potassium phosphate, pH 7.0; 1 mM 2 mercaptoethanol (2-ME); and 0.5 mM EDTA). The cell free extract was obtained by centrifugation at 24,000 x g for 30 minutes. Protamine sulfate was added to the extract at a final concentration of 2.0 mg/ml and the mixture was stirred for 1 hour. The precipitates formed were removed by centrifugation. The supernatant was used as the cell-free extract in the following isolation procedure.

### B. Isolation of Crude PNB esterase

The extract was subjected to ammonium sulfate fractionation. The precipitates formed between 45-80% ammonium sulfate saturation were collected by centrifugation and dissolved in Buffer A, and then dialyzed overnight against the same buffer. The dialyzed sample was acidified to pH 5.0 with 1 N acetic acid, incubated for 10 minutes and subjected to centrifugation to remove the precipitates. The supernatant contained the crude PNB esterase.

### C. Purification of PNB esterase

The supernatant was adjusted to pH 8.5 with 2 N NH₄OH an applied onto a weak anionic resin (DE52 column, 3.7 x 35 cm; Pharmacia, Inc., Piscataway, NJ 08854) equilibrated with Buffer B (10 mM Tris-HCl, pH 8.5; 50 mM NaCl; 1 mM 2-ME; and 0.5 mM EDTA). The column was washed with 350 ml of Buffer B and again with 1,750 ml of Buffer C (10 mM Tris-HCl, pH 7.0; 50 mM NaCl; 1 mM 2-ME; and 0.5 mM EDTA). The PNB esterase was eluted with a 3,500 ml gradient of 50-300 mM NaCl in Buffer C. Fractions of the PNB esterase were pooled and concentrated by ultrafiltration with an AMICON PM-10 membrane (Amicon, Danvers, MA 01923). The concentrated crude PNB esterase solution was subjected to gel filtration employing a polysaccharide-type gel (Sephacryl S-200 HR column, 5.0 x 95 cm; Pharmacia, Inc.) equilibrated with Buffer D (10 mM Tris-HCl, pH 8.0; 1 mM 2-ME; and 0.5 mM EDTA). The fractions containing enzyme activity were combined, concentrated by ultrafiltration and dialyzed overnight against Buffer B. The dialyzed solution was applied onto an anionic exchange resin, (Q-Sepharose column, 2.6 x 20 cm; Pharmacia Inc,) equilibrated with Buffer B. The column was washed with 100 ml of Buffer B and subsequently with 500 ml of Buffer E (10 mM MES-NaOH, pH 6.0; 10 mM NaCl; 1 mM 2-ME; and 0.5 mM EDTA). The PNB esterase was eluted with a 1,500 ml linear gradient of 100-300 mM NaCl in Buffer E. The fractions containing the PNB esterase were combined, concentrated by ultrafiltration, and dialyzed overnight against 10 mM sodium acetate, pH 5.0, containing 1 mM 2-ME and 0.5 mM EDTA.

After removing the precipitate by centrifugation, the enzyme solution was loaded onto a calcium phosphate-cellulose column (1.6 x 20) equilibrated with a buffer containing 10 mM sodium acetate, pH 5.0, containing 1 mM 2-ME and 0.5 mM EDTA. The finely-divided calcium phosphate used can be prepared according to Jenner, U.S. Pat. No. 3,737,516. After the column was washed with 250 ml of the same buffer, the PNB esterase was eluted with a 250 ml linear gradient of 10-50 mM potassium phosphate, pH 7.0, containing 1 mM 2-ME and 0.5 mM EDTA. The fractions containing the PNB esterase activity were pooled, concentrated by ultrafiltration, and then dialyzed overnight against Buffer D.

The dialyzed enzyme solution was applied onto a *p*-aminobenzamidine-agarose column (1.0 x 20 cm; Pharmacia Inc.) equilibrated with Buffer D. After washing the column with 50 ml of Buffer D, the PNB esterase was eluted with 100 ml linear gradient of 0-300 mM NaCl in Buffer D. The fractions with the PNB esterase activity eluted approximately between 160 and 220 mM NaCl in Buffer D were pooled, concentrated by ultrafiltration, and represented the purified enzyme.

All steps of the above-described purification were performed at temperatures between 0-4°C. The progress of the purification was followed by assaying the material for PNB esterase activity obtained in each step of the isolation and purification.

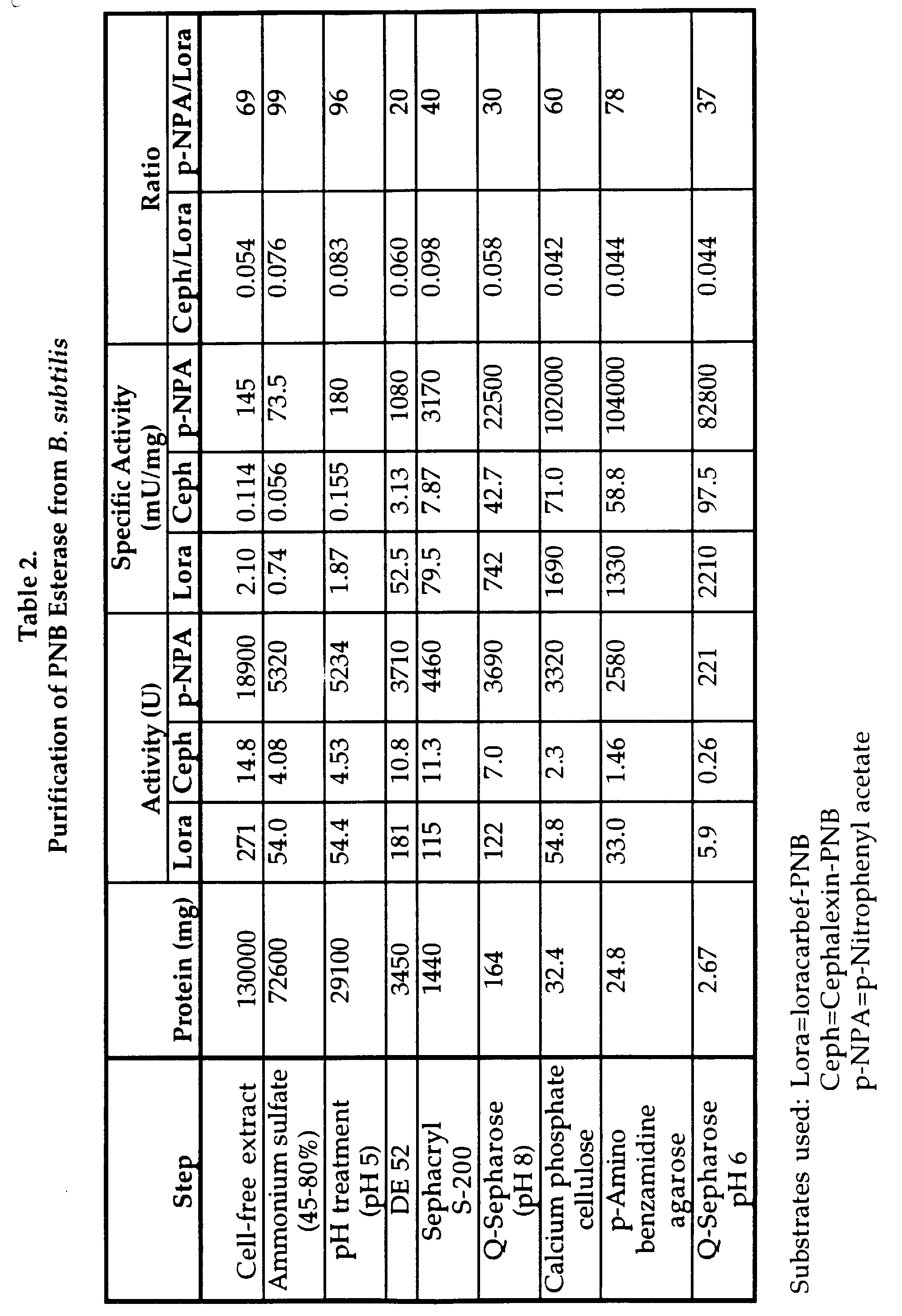

### D. Assay Method for PNB Esterase Activity

A 1 ml reaction mixture containing 5 µmoles Bis-Tri-Propane-HCl, pH 6.5, 0.5 µmole substrate (loracarbef PNB ester or cefaclor PNB ester), and appropriate amounts of enzyme solution was incubated at 30°C in a constant-temperature shaker for 30 min. The reaction was stopped by the addition of an equal volume of acetonitrile. The mixture was then centrifuged to remove the protein and the supernatant solution was analyzed by high performance liquid chromatography (HPLC) for product formation and substrate disappearance. The HPLC was performed in a C-18 reverse-phase column (Beckman Ultrosphere ODS, Beckman Instruments Inc., Fullerton, CA 92634) with a linear gradient formed by Buffer A containing 80% of 1 mM Triethylamine-HCl, pH 2.5 and 20% methanol and Buffer B containing methanol, at a flow rate of 1 ml/min.

The HPLC system used was a Varian HPLC system including a Vista 5560 unit (Varian Associates, Sugar Land TX) and a model 230-401 auto-sampling injector (Gilson Medical Electronics, Middleton, WI.). The PNB esterase activity was determined by monitoring product formation at 254 nm.

Taking advantage of the absorption change in the hydrolysis of *p*-nitrophenylacetate to *p*-nitrophenyl alcohol and acetate, a spectrophotometric assay for PNB esterase was developed. An enzyme fraction showing activity in this assay may not necessarily have the PNB esterase activity, but the PNB esterase will show activity in this assay. For the purified PNB esterase, the results of this assay correlated valuable in the purification of-the enzyme,where large numbers of assays are generally involved. This-assay was carried out at room temperature in a 1 ml assay mixture, containing 100 µM Tris-HCl (pH 7.0), 1.6 µM *p*-nitrophenylacetate and 1-20 µl enzyme solution. The activity was followed by measuring the absorption change at 405 nm in either a Cary spectrophotometer, model 219 or Beckman DU-50.

The following SEQUENCE LISTING provides the DNA and amino acid sequences of compounds of the present invention. These sequences are referred to in the specification and claims by SEQ. ID. NO. The ordinarily skilled artisan will recognize that the DNA sequences are listed in the 5' to 3' direction, and the amino acid sequences are lised from the amino terminus to the carboxy terminus. The amino acid residues are designated as follows: Ala is an alanine residue, Arg is an arginine residue, Asn is an asparagine residue, Asp is an aspartic acid residue, Cys is a cysteine residue, Gln is a glutamine residue, Glu is a glutamic acid residue, Gly is a glycine residue, His is a histidine residue, Ile is an isoleucine residue, Leu is a leucine residue, Lys is a lysine residue, Met is a methionine residue, Phe is a phenylalanine residue, Pro is a proline residue, Ser is a serine residue, Thr is a threonine residue, Trp is a tryptophan residue, Tyr is a tyrosine residue, and Val is a valine residue. In the DNA sequences A is deoxyadenyl, G is deoxyguanyl, C is deoxycytidyl, and T is thymidyl.

## Claims

1. An isolated DNA compound that comprises a DNA sequence encoding PNB esterase activity of *Bacillus subtilis*.

2. An isolated DNA compound of Claim 1 that encodes a protein of SEQ. ID. NO: 2 as hereinbefore described.

3. An isolated DNA compound of Claim 2 wherein the coding strand comprises SEQ. ID. NO: 1 as hereinbefore described.

4. A recombinant DNA vector that comprises a DNA sequence as claimed in any one of Claims 1 to 3.

5. A recombinant DNA vector of Claim 4 that further comprises a promoter positioned to drive expression of said PNB esterase activity encoding DNA.

6. A recombinant DNA vector of Claim 5 wherein said promoter functions in *Escherichia coli*.

7. A host cell transformed with a recombinant DNA vector as claimed in any one of Claims 4 to 6.

8. An *Escherichia coli* cell transformed with a recombinant DNA vector as claimed in any one of Claims 4 to 6.

9. A method for constructing a recombinant host cell capable of expressing PNB esterase activity, said method comprising:
transforming said host cell with a recombinant DNA expression vector that comprises:
(a) a promoter and translational activating sequence that functions in said host cell
(b) a DNA sequence encoding PNB esterase activity of *Bacillus subtilis* positioned for the expression from said promoter and translational activating sequence.

10. A method for expressing PNB esterase activity comprising culturing a host cell transformed with a recombinant DNA expression vector that comprises:
(a) a promoter and translational activating sequence that functions in said host cell;
(b) a DNA sequence encoding PNB esterase activity of *Bacillus subtilis* positioned for the expression from said promoter and translational activating sequence;
under conditions suitable for gene expression.

11. A method of Claim 11 wherein said recombinant DNA expression vector comprises a DNA sequence as claimed in any one of Claims 2 or 3.

12. A method of Claim 11 wherein said host cell is *Escherichia coli*.

13. A process for the removal of the *p*-nitrobenzyl group from a cephalosporin PNB ester or a 1-carbacephalosporin PNB ester which comprises reacting said ester with a PNB esterase prepared by the process of Claim 10, 11 or 12.

## Patentansprüche

1. Isolierte DNA Verbindung, die eine DNA Sequenz enthält, welche eine PNB Esteraseaktivität von *Bacillus subtilis* kodiert.

2. Isolierte DNA Verbindung nach Anspruch 1, die ein Protein der SEQ ID Nr:2 kodiert, wie dies hierin vorher beschrieben wurde.

3. Isolierte DNA Verbindung nach Anspruch 2, worin der kodierende Strang die SEQ ID Nr:1 enthält, wie dies vorher beschrieben wurde.

4. Rekombinanter DNA Vektor, der eine DNA Sequenz nach einem der Ansprüche 1 bis 3 enthält.

5. Rekombinanter DNA Vektor nach Anspruch 4, der ferner einen Promotor enthält, welcher zur Expressionssteuerung dieser für PNB Esterase kodierenden DNA positioniert ist.

6. Rekombinanter DNA Vektor nach Anspruch 5, worin dieser Promotor in *Escherichia coli* funktionsfähig ist.

7. Wirtszelle, die mit einem rekombinanten DNA Vektor nach einem der Ansprüche 4 bis 6 transformiert ist.

8. *Escherichia coli* Zelle, die mit einem rekombinanten DNA Vektor nach einem der Ansprüche 4 bis 6 transformiert ist.

9. Verfahren zur Konstruktion einer rekombinanten Wirtszelle, die zur Expression der PNB Esteraseaktivität fähig ist, gekennzeichnet durch
Transformation dieser Wirtszelle mit einem rekombinanten DNA Expressionsvektor, der umfaßt
(a) einen Promotor und eine Translationsaktivierungssequenz, die in dieser Wirtszelle funktionsfähig sind
(b) eine DNA Sequenz, die für eine PNB Esteraseaktivität von *Bacillus subtilis* kodiert und zur Expression von diesem Promotor und dieser Translationsaktivierungssequenz positioniert ist.

10. Verfahren zur Expression der PNB Esteraseaktivität, gekennzeichnet durch Kultivierung einer Wirtszelle, die mit einem rekombinanten DNA Expressionsvektor transformiert ist, der umfaßt
(a) einen Promotor und eine Translationsaktivierungssequenz, die in dieser Wirtszelle funktionsfähig sind
(b) eine DNA Sequenz, die für eine PNB Esteraseaktivität von *Bacillus subtilis* kodiert und zur Expression von diesem Promotor und dieser Translationsaktivierungssequenz positioniert ist,
unter zur Genexpression geeigneten Bedingungen.

11. Verfahren nach Anspruch 10, worin dieser rekombinante DNA Expressionsvektor eine DNA Sequenz nach einem der Ansprüche 2 oder 3 umfaßt.

12. Verfahren nach Anspruch 10, worin diese Wirtszelle *Escherichia coli* ist.

13. Verfahren zur Entfernung der p-Nitrobenzylgruppe von einem Cephalosporin-PNB-ester oder einem 1-Carbacephalosporin-PNB-ester, gekennzeichnet durch Umsetzung dieses Esters mit einer PNB Esterase, die durch das Verfahren nach Anspruch 10, 11 oder 12 hergestellt wurde.

## Revendications

1. Composé d'ADN isolé qui comprend une séquence d'ADN encodant l'activité de PNB estérase de *Bacillus subtilis.*

2. Composé d'ADN isolé selon la revendication 1, qui encode une protéine de SEQ. ID. NO: 2 telle que décrite ci-dessus.

3. Composé d'ADN isolé selon la revendication 2, dans lequel le brin codant comprend SEQ. ID. NO: 1 telle que décrite ci-dessus.

4. Vecteur d'ADN recombiné qui comprend une séquence d'ADN selon l'une quelconque des revendications 1 à 3.

5. Vecteur d'ADN recombiné selon la revendication 4, qui comprend en outre un promoteur positionné pour guider l'expression dudit ADN encodant l'activité de PNB estérase.

6. Vecteur d'ADN recombiné selon la revendication 5, dans lequel ledit promoteur fonctionne dans *Escherichia coli.*

7. Cellule hôte transformée avec un vecteur d'ADN recombinant selon l'une quelconque des revendications 4 à 6.

8. Cellule de *Escherichia coli* transformée avec un vecteur d'ADN recombiné selon l'une quelconque des revendications 4 à 6.

9. Procédé pour construire une cellule hôte recombinée capable d'exprimer l'activité de PNB estérase, ledit procédé comprenant le fait de:
transformer ladite cellule hôte avec un vecteur d'expression d'ADN recombiné qui comprend:
(a) une séquence de promoteur et d'activation de traduction qui fonctionne dans ladite cellule hôte;
(b) une séquence d'ADN encodant l'activité de PNB estérase de *Bacillus subtilis,* positionnée pour l'expression à partir de ladite séquence de promoteur et d'activation de traduction.

10. Procédé pour exprimer l'activité de PNB estérase, comprenant la mise en culture d'une cellule hôte transformée avec un vecteur d'expression d'ADN recombiné qui comprend:
(a) une séquence de promoteur et d'activation de traduction qui fonctionne dans ladite cellule hôte;
(b) une séquence d'ADN encodant l'activité de PNB estérase de Bacillus subtilis, positionnée pour l'expression à partir de ladite séquence de promoteur et d'activation de traduction;
dans des conditions appropriées pour l'expression génique.

11. Procédé selon la revendication 1, dans lequel ledit vecteur d'expression d'ADN recombiné comprend une séquence d'ADN selon l'une quelconque des revendications 2 ou 3.

12. Procédé selon la revendication 11, dans lequel ladite cellule hôte est *Escherichia coli.*

13. Procédé pour l'élimination du groupe p-nitrobenzyle d'un ester PNB de céphalosporine ou d'un ester PNB de 1-carbacéphalosporine, qui comprend la mise en réaction dudit ester avec une PNB estérase préparée par le procédé selon les revendications 10, 11 ou 12.


### SEQUENCE LISTING

(1) GENERAL INFORMATION:
   (i) APPLICANT: Eli Lilly and Company
   (ii) TITLE OF INVENTION: Recombinant DNA Compounds and Expression Vectors Encoding para-Nitrobenzyl Esterase Activity of Bacillus
   (iii) NUMBER OF SEQUENCES: 7
   (iv) CORRESPONDENCE ADDRESS:
      (A) ADDRESSEE: Eli Lilly and Company
      (B) STREET: Erl Wood Manor
      (C) CITY: Windlesham
      (D) STATE: Surrey
      (E) COUNTRY: United Kingdom
      (F) ZIP: GU20 6PH
   (viii) ATTORNEY/AGENT INFORMATION:
      (A) NAME: Hudson, Christopher Mark
      (B) REGISTRATION NUMBER: GA307
      (C) REFERENCE/DOCKET NUMBER: X-8315
   (ix) TELECOMMUNICATION INFORMATION:
      (A) TELEPHONE: (0276) 478441
      (B) TELEFAX: (0276) 478306
(2) INFORMATION FOR SEQ ID NO:1:
   (i) SEQUENCE CHARACTERISTICS:
      (A) LENGTH:1470 nucleotides
      (B) TYPE:nucleic acid
      (C) STRANDEDNESS:double
      (D) TOPOLOGY:linear
   (ii) MOLECULE TYPE:DNA
   (xi) SEQUENCE DESCRIPTION:SEQ ID NO:1
   ATG ACT CAT CAA ATA GTA ACG ACT CAA TAC GGC AAA GTA AAA 42
   GGC ACA ACG GAA AAC GGC GTA CAT AAG TGG AAA GGC ATC CCC 84
   TAT GCC AAG CCG CCT GTC GGA CAA TGG CGT TTT AAA GCA CCT 126
   GAG CCG CCT GAA GTG TGG GAA GAT GTC CTT GAT GCC ACA GCG 168
   TAC GGT CCT ATT TGC CCG CAG CCG TCT GAT TTG CTC TCA CTG 210
   TCG TAT ACA GAG CTG CCC CGC CAG TCC GAG GAT TGC TTG TAT 252
   GTC AAT GTA TTT GCG CCT GAC ACT CCA AGT CAA AAT CTT CCT 294
   GTC ATG GTG TGG ATT CAC GGA GGC GCT TTT TAT CTT GGA GCG 336
   GGC AGT GAG CCA TTG TAT GAC GGA TCA AAA CTT GCG GCA CAG 378
   GGA GAA GTC ATT GTC GTT ACA TTG AAC TAT CGG CTG GGG CCG 420
   TTT GGC TTT TTG CAC TTG TCT TCG TTT GAT GAG GCG TAT TCC 462
   GAT AAC CTT GGG CTT TTA GAC CAA GCC GCC GCG CTG AAA TGG 504
   GTG CGG GAG AAT ATC TCA GCG TTT GGC GGT GAT CCC GAT AAC 546
   GTA ACA GTA TTT GGA GAA TCC GCC GGC GGC ATG AGC ATT GCC 588
   GCG CTG CTC GCT ATG CCT GCG GCA AAA GGC CTG TTC CAG AAA 630
   GCG ATC ATG GAA AGC GGC GCT TCC CGA ACA ATG ACA AAA GAA 672
   CAA GCG GCA AGC ACT GCG GCT GCC TTT TTA CAG GTC CTT GGG 714
   ATT AAT GAG AGC CAG CTG GAC AGA TTG CAT ACT GTA GCA GCG 756
   GAA GAT TTG CTT AAA GCG GCC GAT CAG CTT CGG ATT GCA GAA 798
   AAA GAA AAT ATC TTT CAG CTG TTC TTC CAG CCC GCC CTT GAT 840
   CCG AAA ACG CTG CCT GAA GAA CCA GAA AAA TCG ATC GCA GAA 882
   GGG GCT GCT TCC GGC ATT CCG CTA TTG ATT GGA ACA ACC CGT 924
   GAT GAA GGA TAT TTA TTT TTC ACC CCG GAT TCA GAC GTT CAT 966
   TCT CAG GAA ACG CTT GAT GCA GCA CTC GAG TAT TTA CTA GGG 1008
   AAG CCG CTG GCA GAG AAA GCT GCC GAT TTG TAT CCG CGT TCT 1050
   CTG GAA AGC CAA ATT CAT ATG ATG ACT GAT TTA TTA TTT TGG 1092
   CGC CCT GCC GTC GCC TAT GCA TCC GCA CAG TCT CAT TAC GCC 1134
   CCT GTC TGG ATG TAC CGG TTC GAT TGG CAC CCG GAG AAG CCG 1176
   CCG TAC AAT AAA GCG TTT CAC GCA TTA GAG CTT CCT TTT GTC 1218
   TTT GGA AAT CTG GAC GGA TTG GAA CGA ATG GCA AAA GCG GAG 1260
   ATT ACG GAT GAG GTG AAA CAG CTT TCT CAC ACG ATA CAA TCC 1302
   GCG TGG ATC ACG TTC GCT AAA ACA GGA AAC CCA AGC ACC GAA 1344
   GCT GTG AAT TGG CCG GCG TAT CAT GAA GAA ACG AGA GAG ACG 1386
   GTG ATT TTA GAC TCA GAG ATT ACG ATC GAA AAC GAT CCC GAA 1428
   TCT GAA AAA AGG CAG AAG CTA TTC CCT TCA AAA GGA GAA TAA 1470
(2) INFORMATION FOR SEQ ID NO:2:
   (i) SEQUENCE CHARACTERISTICS:
      (A) LENGTH:489 amino acids
      (B) TYPE:amino acid
      (C) STRANDEDNESS:single
      (D) TOPOLOGY:linear
   (ii) MOLECULE TYPE:protein
   (xi) SEQUENCE DESCRIPTION:SEQ ID NO:2
   Met Thr His Gln Ile Val Thr Thr Gln Tyr Gly Lys Val Lys Gly
    1               5                  10                  15
   Thr Thr Glu Asn Gly Val His Lys Trp Lys Gly Ile Pro Tyr Ala
                   20                  25                  30
   Lys Pro Pro Val Gly Gln Trp Arg Phe Lys Ala Pro Glu Pro Pro
                   35                  40                  45
   Glu Val Trp Glu Asp Val Leu Asp Ala Thr Ala Tyr Gly Pro Ile
                   50                  55                  60
   Cys Pro Gln Pro Ser Asp Leu Leu Ser Leu Ser Tyr Thr Glu Leu
                   65                  70                  75
   Pro Arg Gln Ser Glu Asp Cys Leu Tyr Val Asn Val Phe Ala Pro
                   80                  85                  90
   Asp Thr Pro Ser Gln Asn Leu Pro Val Met Val Trp Ile His Gly
                   95                 100                 105
   Gly Ala Phe Tyr Leu Gly Ala Gly Ser Glu Pro Leu Tyr Asp Gly
                       110                           115                           120
   Ser Lys Leu Ala Ala Gln Gly Glu Val Ile Val Val Thr Leu Asn
                       125                           130                           135
   Tyr Arg Leu Gly Pro Phe Gly Phe Leu His Leu Ser Ser Phe Asp
                       140                           145                           150
   Glu Ala Tyr Ser Asp Asn Leu Gly Leu Leu Asp Gln Ala Ala Ala
                       155                           160                           165
   Leu Lys Trp Val Arg Glu Asn Ile Ser Ala Phe Gly Gly Asp Pro
                       170                           175                           180
   Asp Asn Val Thr Val Phe Gly Glu Ser Ala Gly Gly Met Ser Ile
                       185                           190                           195
   Ala Ala Leu Leu Ala Met Pro Ala Ala Lys Gly Leu Phe Gln Lys
                       200                           205                           210
   Ala Ile Met Glu Ser Gly Ala Ser Arg Thr Met Thr Lys Glu Gln
                       215                           220                           225
   Ala Ala Ser Thr Ala Ala Ala Phe Leu Gln Val Leu Gly Ile Asn
                       230                           235                           240
   Glu Ser Gln Leu Asp Arg Leu His Thr Val Ala Ala Glu Asp Leu
                       245                           250                           255
   Leu Lys Ala Ala Asp Gln Leu Arg Ile Ala Glu Lys Glu Asn Ile
                       260                           265                           270
   Phe Gln Leu Phe Phe Gln Pro Ala Leu Asp Pro Lys Thr Leu Pro
                       275                           280                           285
   Glu Glu Pro Glu Lys Ser Ile Ala Glu Gly Ala Ala Ser Gly Ile
                       290                           295                           300
   Pro Leu Leu Ile Gly Thr Thr Arg Asp Glu Gly Tyr Leu Phe Phe
                       305                           310                           315
   Thr Pro Asp Ser Asp Val His Ser Gln Glu Thr Leu Asp Ala Ala
                       320                           325                           330
   Leu Glu Tyr Leu Leu Gly Lys Pro Leu Ala Glu Lys Ala Ala Asp
                       335                           340                           345
   Leu Tyr Pro Arg Ser Leu Glu Ser Gln Ile His Met Met Thr Asp
                       350                           355                           360
   Leu Leu Phe Trp Arg Pro Ala Val Ala Tyr Ala Ser Ala Gln Ser
                       365                           370                           375
   His Tyr Ala Pro Val Trp Met Tyr Arg Phe Asp Trp His Pro Glu
                       380                           385                           390
   Lys Pro Pro Tyr Asn Lys Ala Phe His Ala Leu Glu Leu Pro Phe
                       395                           400                           405
   Val Phe Gly Asn Leu Asp Gly Leu Glu Arg Met Ala Lys Ala Glu
                       410                           415                           420
   Ile Thr Asp Glu Val Lys Gln Leu Ser His Thr Ile Gln Ser Ala
                       425                           430                           435
   Trp Ile Thr Phe Ala Lys Thr Gly Asn Pro Ser Thr Glu Ala Val
                       440                           445                           450
   Asn Trp Pro Ala Tyr His Glu Glu Thr Arg Glu Thr Val Ile Leu
                       455                           460                           465
   Asp Ser Glu Ile Thr Ile Glu Asn Asp Pro Glu Ser Glu Lys Arg
                       470                           475                           480
   Gln Lys Leu Phe Pro Ser Lys Gly Glu
                       485                 489
(2) INFORMATION FOR SEQ ID NO:3:
   (i) SEQUENCE CHARACTERISTICS:
      (A) LENGTH:22 amino acids
      (B) TYPE:amino acid
      (C) STRANDEDNESS:single
      (D) TOPOLOGY:linear
   (ii) MOLECULE TYPE:peptide
   (xi) SEQUENCE DESCRIPTION:SEQ ID NO:3
   Met Thr His Gln Ile Val Thr Thr Tyr Gly Lys Lys Val Lys Gly
                    5                  10                  15
   Thr Gln Glu Asn Gly Val His
                   20
(2) INFORMATION FOR SEQ ID NO:4:
   (i) SEQUENCE CHARACTERISTICS:
      (A) LENGTH:41 nucleotides
      (B) TYPE:nucleic acid
      (C) STRANDEDNESS:single
      (D) TOPOLOGY:linear
   (ii) MOLECULE TYPE:DNA
   (xi) SEQUENCE DESCRIPTION:SEQ ID NO:4
   ATGACACATC AAATTGTCAC AACATATGGC AAAAAAGTCA A 41
(2) INFORMATION FOR SEQ ID NO:5:
   (i) SEQUENCE CHARACTERISTICS:
      (A) LENGTH:41 nucleotides
      (B) TYPE:nucleic acid
      (C) STRANDEDNESS:single
      (D) TOPOLOGY:linear
   (ii) MOLECULE TYPE:DNA
   (xi) SEQUENCE DESCRIPTION:SEQ ID NO:5
   TATGGCAAAA AAGTCAAAGG CACACAAGAA AATGGCGTCC A 41
(2) INFORMATION FOR SEQ ID NO:6:
   (i) SEQUENCE CHARACTERISTICS:
      (A) LENGTH:34 nucleotides
      (B) TYPE:nucleic acid
      (C) STRANDEDNESS:single
      (D) TOPOLOGY:linear
   (ii) MOLECULE TYPE:DNA
   (xi) SEQUENCE DESCRIPTION:SEQ ID NO:6
   AAAAAGGGAG AGAACCATAT GACTCATCAA ATAG 34
(2) INFORMATION FOR SEQ ID NO:7:
   (i) SEQUENCE CHARACTERISTICS:
      (A) LENGTH:20 nucleotides
      (B) TYPE:nucleic acid
      (C) STRANDEDNESS:single
      (D) TOPOLOGY:linear
   (ii) MOLECULE TYPE:DNA
   (xi) SEQUENCE DESCRIPTION:SEQ ID NO:7
   TTGACATACA AGCAATCCTC 20